Europäisches Patentamt

European Patent Office

Office européen des brevets

(19)

(11) Veröffentlichungsnummer: **0 312 022 B1**

(12) **EUROPÄISCHE PATENTSCHRIFT**

(45) Veröffentlichungstag der Patentschrift:
24.07.91 Patentblatt 91/30

(51) Int. Cl.⁵: **A61L 2/08, B65B 55/08**

(21) Anmeldenummer: 88116951.0

(22) Anmeldetag: 12.10.88

(54) **Verfahren zur Sterilisation von temperaturbelastbaren Behältern unter Reinraumbedingungen.**

(30) Priorität: 14.10.87 DE 3734830

(43) Veröffentlichungstag der Anmeldung:
19.04.89 Patentblatt 89/16

(45) Bekanntmachung des Hinweises auf die
Patenterteilung:
24.07.91 Patentblatt 91/30

(84) Benannte Vertragsstaaten:
CH DE FR GB IT LI NL

(56) Entgegenhaltungen:
DE-A- 2 631 352
DE-A- 3 321 195
DE-A- 3 510 286
FR-A- 2 547 732
US-A- 3 676 058

(73) Patentinhaber: HANS GILOWY
MASCHINENFABRIK METEORWERK GmbH &
CO.
Schmalenbachstrasse 12-16
W-1000 Berlin 44 (DE)

(72) Erfinder: Reiss, Roland
Siemensstrasse 10
W-1000 Berlin 21 (DE)
Erfinder: Liskow, Gerd
Königsbacher Zeile 39
W-1000 Berlin 28 (DE)
Erfinder: Gehrke, Hinrich
Zadekstrasse 16a
W-1000 Berlin 47 (DE)

Erfinder: Luxner, Walter
Schliesserstrasse 27
W-1000 Berlin 27 (DE)
Erfinder: Schmidt, Gerd
Forststrasse 9
W-1000 Berlin 41 (DE)
Erfinder: Winiarski, Dieter
Zweibrücker Strasse 17b
W-1000 Berlin 20 (DE)
Erfinder: Lindner, Eckehart
Adolf-Kolping-Strasse 16
W-5068 Odenthal-Hahnenberg (DE)
Erfinder: Sirch, Edgar
Heinrich-von-Brentano-Strasse 4
W-5090 Leverkusen 1 (DE)
Erfinder: Kalbfleisch, Eckhard
Carl-Rumpff-Strasse 5
W-5090 Leverkusen 1 (DE)
Erfinder: Laubert, Hans Peter
Ophovener Strasse 15a
W-5090 Leverkusen 1 (DE)
Erfinder: Kiefer, Peter
Wingensiefener Strasse 24
W-5068 Odenthal-Glöbusch (DE)
Erfinder: Franz, Johann
Robert-Koch-Strasse 34
W-5090 Leverkusen 3 (DE)
Erfinder: Fasse, Anton
Auf dem Acker 3
W-5024 Pulheim 5 (DE)

(74) Vertreter: Wey, Hans-Heinrich, Dipl.-Ing.
Patentanwälte Wey & Partner
Widenmayerstrasse 49
W-8000 München 22 (DE)

## Beschreibung

Die Erfindung geht aus von einem Verfahren zur Trockenhitze-Sterilisation von temperaturbelastbaren Behältern unter Reinraumbedingungen, insbesondere Glasflaschen zur Abfüllung pharmazeutischer Produkte, bei dem die Behälter kontinuierlich über Laminar-Flow-Einheiten in einen Strahlungsofen ein- und ausgeschleust und anschließend durch HOSCH-filtrierte Luft abgekühlt werden, wobei ein Teil der HOSCH-filtrierten Luft am Ausgang des Strahlungsofens abgezweigt wird und in einer turbulenzarmen und geregelten Verdrängungsströmung durch den Strahlungsofen hindurchgeleitet wird.

Ein Verfahren dieser Art mit dem dazugehörigen Strahlungsofen ist in dem deutschen Patent 26 31 352 beschrieben. Das hierin beschriebene Sterilisationsverfahren hat sich im großen und ganzen bewährt, wenn hinsichtlich der maximal zulässigen Partikelzahl Klasse 100, US-Fed. Standard 209 b (bzw. etwa Klasse 3 VDI 2083) niedergelegten Forderungen eingehalten werden soll.

Die neue Ausgabe dieses auch für die pharmazeutische Fertigung relevanten Standards (US-Fed. Standard 209 c) definiert darüber hinaus noch die höherwertige Klasse 10, die mit dem beschriebenen Strahlungsofen erreicht werden soll.

Ausgehend von dieser Zielsetzung liegt der Erfindung die Aufgabe zugrunde, das oben beschriebene Trockenhitze-Sterilisierverfahren unter Verwendung eines nach dem Prinzip der turbulenzarmen Verdrängerströmung arbeitenden Strahlungsofens noch weiter zu verbessern.

Diese Aufgabe wird, ausgehend von dem eingangs beschriebenen Verfahren, erfindungsgemäß dadurch gelöst, daß die Reinraumbedingungen im Strahlungsofen durch einen Überdruck $P_2$ gegenüber dem Atmosphärendruck $P_{AT}$ und eine daraus resultierende Gegenströmung mit einer mittleren Strömungsgeschwindigkeit unterhalb 0,2 m/s aufrechterhalten werden, wobei der Überdruck $P_2$ im Strahlungsofen gemessen und mit einem Sollwert verglichen wird und der ausgangsseitige Abluftventilator für die HOSCH-filtrierte Kühlluft derart nachgeregelt wird, daß die Abweichung $\Delta p$ vom Sollwert minimiert wird, und daß der größte Teil der — vorzugsweise sowohl über als auch unter den Flaschen angeordneten — Strahlungsheizelemente mit konstanter elektrischer Leistung betrieben wird und mit dem verbleibenden Teil der Heizelemente die durch instationäre Betriebszustände verursachten Temperaturschwankungen weggeregelt werden. Dabei sind sämtliche Druckangaben als Überdruck gegenüber dem Atmosphärendruck $P_{AT}$ zu verstehen.

Aufgrund dieser kombinierten Maßnahmen gelingt es, im Strahlungsofen während der gesamten Betriebsdauer eine ungestörte, turbulenzarme Verdrängungsströmung aufrechtzuerhalten. Es hat sich herausgestellt, daß der Druck im Strahlungsofen als empfindliche und besonders gut zu verarbeitende Meßgröße für die Einhaltung konstanter Strömungsbedingungen bei derart geringen Strömungsgeschwindigkeiten ( < 0,2 m/s) benutzt werden kann. Die Aufteilung der Heizleistung bei den Strahlungsheizelementen auf eine anteilig überwiegende konstante Last (Grundlast) und eine dagegen wesentlich kleinere Regellast sorgt dafür, daß die die turbulenzarme Gegenströmung störenden Konvektionsströmungen auf das technisch mögliche Minimum reduziert werden.

Vorzugsweise wird der Sollwert für den Überdruck $P_2$ im Strahlungsofen auf einen Wert $\geq 0,2$ Pa eingestellt. Hinsichtlich der Strömungsgeschwindigkeit im Strahlungsofen mit einem freien Querschnitt von ca. 0,7 m² wird dabei eine mittlere Reynolds-Zahl unterhalb des kritischen Wertes von 2300 eingehalten.

Gemäß einer Weiterbildung der Erfindung wird die Reinraumklasse im Strahlungsofen mit Hilfe von rasterartig angeordneten, nacheinander auf einen Partikelzähler aufschaltbaren Ansaugtulpen oberhalb der Glasbehälter kontinuierlich überwacht und dokumentiert. Auf diese Weise kann die Reinraumklasse während des gesamten Betriebes zuverlässig überwacht werden.

Zweckmäßigerweise wird außerdem die Sterilisationstemperatur der Glasbehälter pyrometrisch direkt gemessen und bei Unterschreitung einer vorgegebenen Solltemperatur das Transportband angehalten, so daß die Glasbehälter sicher auf die Sterilisationstemperatur gebracht werden können.

Um auch beim An- bzw. Abfahren des Strahlungsofens die Reinraumbedingungen aufrechtzuerhalten, werden die Glasbehälter vorteilhaft in dichter Packung auf dem Transportband durch den Strahlungsofen gefahren und beim Füllen und Leerfahren des Strahlungsofens die Blenden am Aus- bzw. Eingang derart nachgeregelt, daß die zu den stationären Reinraumbedingungen gehörenden Druck- und Temperaturwerte im Strahlungsofen weitestgehend konstant bleiben.

Die Verdrängungsströmung störende Konvektionsströmungen können außerdem minimiert werden, wenn die Temperatur über die volle Breite des Strahlungsofens durch zusätzliche, in die Seitenwände integrierte Heizelemente vergleichmäßigt wird. Damit werden auch die Temperaturabweichungen am Sterilisiergut verringert.

Unmittelbar am Austritt der Flaschen aus dem Strahlungsofen herrscht ein relativ großer Drucksprung zwischen dem Druckniveau $P_2$ des Strahlungsofens und dem in der als Kühlzone ausgebildeten Laminar-Flow-Schleuse vorherrschenden Druck $P_3$. Durch diesen Drucksprung wird ein Injektionsstrom mit relativ großer Strömungsgeschwindigkeit erzeugt, der zu einer Störung der turbulenzarmen Verdrängungsströmung in diesem Bereich führt. Eine weitere Verbesserung kann daher erreicht werden, wenn das Druckgefälle zwischen Kühlzone und Strahlungsofen über ein eingebautes Laminarisierungsgewebe zusätzlich abgebaut wird, wodurch die in den Strahlungsofen eintretende Gegenströmung schneller beruhigt und vergleichmäßigt wird.

Mit der Erfindung werden folgende Vorteile erzielt:

1. Systematische Partikelmessungen haben ergeben, daß in dem kritischen Bereich oberhalb der Flaschenöffnungen im Strahlungsofen extrem niedrige Partikelzahlen entsprechend VDI 2083 Klasse 2 aufrechterhalten werden können.

2. Aufgrund der meßtechnischen Validierung und Dokumentation konnte auch nachgewiesen werden, daß die oben angegebenen Reinraumbedingungen während des gesamten Betriebes konstant bleiben.

3. Außerdem können sonstige Störgrößen, wie z.B. durch Temperaturinhomogenitäten verursachte Konvektionsströme, sicher abgefangen werden.

4. Die Regelung der Strömungsgeschwindigkeit der Gegenströmung auf der Basis eines Feindrucksensors im Strahlungsofen läßt sich verhältnismäßig einfach technisch realisieren und bietet den Vorteil einer hohen Empfindlichkeit und Genauigkeit (geringe Regelabweichung).

5. Aufgrund der kontinuierlichen Messung und Dokumentation aller wichtigen Betriebsparameter können die Reinraum- und Sterilisationsbedingungen ständig abgesichert und überwacht werden.

6. Des weiteren kann im Vergleich zum Stand der Technik die Heizleistung im Strahlungsofen durch die starke Verringerung des heißen Verlustmassestroms der Luft noch weiter abgesenkt werden. Das erfindungsgemäße Verfahren erlaubt damit Energieeinsparungen bei Strahlungsöfen in pharmazeutischen Produktionsanlagen.

Im folgenden wird die Erfindung anhand von Zeichnungen und Ausführungsbeispielen näher erläutert. Es zeigen:

Fig. 1    schematisch den Aufbau einer Gesamtanlage zur Strahlungshitzesterilisation von Glasbehältern,
Fig. 2    einen Querschnitt durch den Strahlungshitzetunnel senkrecht zur Transportrichtung der Glasbehälter und
Fig. 3    die Anordnung der Luftansaugstutzen über dem Transportband für die Partikelmessung.

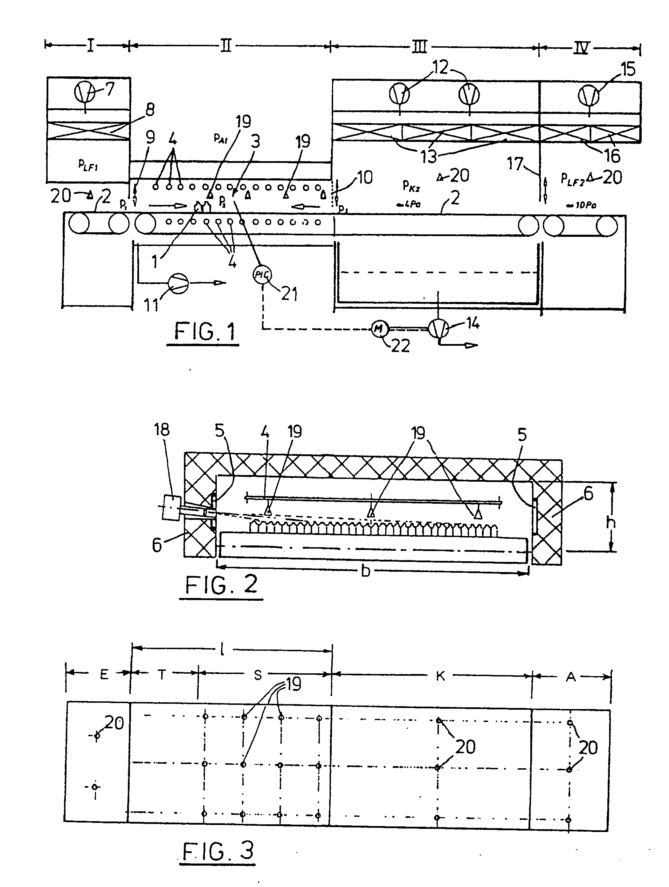

Bei der Anlage zur Durchführung des erfindungsgemäßen Heißsterilisierverfahrens (gemäß Fig. 1) sind vier Zonen vorgesehen, nämlich die Einlaufzone I, die Trocknungs-, Aufheiz- und Sterilisierzone II, die Kühlzone III und die Auslaufzone IV. Die zu sterilisierenden Glasbehälter 1 werden mittels eines Transportbandes durch den die Sterilisierzone II bildenden Strahlungsofen 3 hindurchgeführt. Der Strahlungsofen 3 ist an seiner Ober- und Unter-seite mit stabförmigen IR-Heizelementen 4 bestückt. Außerdem sind parallel zu den Seitenwänden weitere elektrische Heizelemente 5 vorgesehen (siehe Fig. 2), die sich nahezu über die gesamte Länge des Strahlungsofens erstrecken und die Glasbehälter 1 gegenüber den nicht beheizten Seitenwänden 6 des Strahlungsofens thermisch abschirmen. Diese Wandschutzheizung trägt zur Vergleichmäßigung der Temperatur im gesamten Ofenquerschnitt bei und verhindert damit störende seitliche Konvektionsströmungen.

Der Einlauf I besteht aus einer Laminar-Flow-Schleuse mit einem Ventilator 7, der ein Hochleistungsschwebstofffilter 8 (HOSCH-Filter) anströmt und am Eingang des Strahlungshitzetunnels 3 senkrecht zur Transportrichtung einen Luftvorhang erzeugt.

Am Eingang und Ausgang des Strahlungsofens 3 befinden sich jeweils höhenverstellbare Blenden 9 und 10, die so eingestellt werden, daß gerade der Querschnitt des Flaschenpakets am Eintritt bzw. Austritt aus dem Ofen freigegeben wird. Die Blende 10 besteht dabei aus einem Laminarisierungsgewebe, dessen Funktion im folgenden noch erläutert wird. Am Eingang des Strahlungsofens 3, d.h. unterhalb der Eintrittsblende 9, wird die Luft mit einem Wrasenabluft-Ventilator 11 abgesaugt.

In der Kühlzone wird mittels der Ventilatoren 12 und der HOSCH-Filter 13 ebenfalls ein Laminar-Flow-Vorhang erzeugt, der zur Abkühlung der heißen, aus dem Strahlungsofen kommenden Glasbehälter 1 dient. Unterhalb des Transportbandes 2 wird hier die Luft mit dem Abluftventilator 14 abgeführt. An die Kühlzone III schließt sich die Auslaufzone IV an, die wiederum mit einem Ventilator 15 und zwei HOSCH-Filtern 16 bestückt ist. Zwischen Kühlzone III und Auslauf IV ist eine weitere höhenverstellbare Blende 17 angeordnet.

Mit einem Strahlungspyrometer 18, das in eine der Seitenwände 6 eingebaut ist (siehe Fig. 2), kann die Sterilisiertemperatur der Glasbehälter 1 im Strahlungsofen kontinuierlich gemessen und überwacht werden. Mit der Verarbeitung dieses Meßwertes kann z.B. bei einer Unterschreitung der Solltemperatur das Transportband 2 vor Einlauf in die Sterilisierstrecke angehalten und die Behälter 1 auf Sterilisiertemperatur gebracht werden.

Außerdem ist vorgesehen, daß die Partikelzahl im Strahlungsofen direkt gemessen und dokumentiert wird. Zu diesem Zweck sind, wie in Fig. 1, Fig. 2 und Fig. 3 gezeigt, im Strahlungsofen 3, oberhalb der Glasbehälter 1 rasterartig verteilt, eine Vielzahl von Luftansaugstutzen (sogenannte Ansaugtulpen) 19 angeordnet, die nacheinander auf einen Partikelzähler geschaltet werden. Dadurch ist eine kontinuierliche Überwachung und Dokumentation der Reinraumbedingungen im Strahlungsofen gewährleistet.

Zusätzlich können in der Einlauf-, Kühl- und Auslaufzone weitere Ansaugtulpen 20 vorgesehen werden (siehe Fig. 1 und Fig. 3).

Von wesentlicher Bedeutung im Hinblick auf die geforderten extrem niedrigen Partikelzahlen im Strahlungsofen (Reinraumklasse 2 nach VDI-Richtlinie 2083, Blatt 1, Seite 4, Tafel 1) sind die Druckverhältnisse im Strahlungsofen 3 und die daraus resultierenden Strömungsfelder sowie die Temperaturen im Strahlungsofen. In der Einlaufzone I herrscht ein leichter Überdruck $P_{LF1}$ gegenüber dem Atmosphärendruck. Unterhalb der Eintrittsblende 9, wo die Luft durch den Absaugventilator 11 abgesaugt wird, entsteht ein Unterdruck, der mit $P_1$ bezeichnet wird. Im Strahlungsofen 3 herrscht ein Druckgefälle mit einem sehr flachen Druckgradienten, wobei der Druck im Strahlungsofen von links nach rechts, d.h. mit der Transportrichtung zunimmt. Der unterhalb der Austrittsblende 10 herrschende Druck wird mit $P_3$ bezeichnet. Der Druck $P_3$ soll über dem Druck $P_2$ im Strahlungsofen und dem Druck $P_1$ am Eintritt liegen. In der Kühlzone III herrscht ein Druck $P_{KZ}$, der deutlich über dem Druck $P_3$ an der Austrittsblende 10 liegt. Der höchste Druck $P_{LF2}$ wird in der Auslaufzone aufrechterhalten.

Unter diesen Druckbedingungen wird ein kleiner Teil der Luft aus der Kühlzone III abgezweigt und strömt durch die Austrittsblende 10 entgegen der Bewegungsrichtung der Behälter durch den Strahlungsofen hindurch bis zur Abzugsstelle durch den Wrasenabluft-Ventilator 11 abgesaugt wird. Das Druckgefälle zwischen Strahlungs ofen und Kühlzone wird so eingestellt, daß die erwähnte Gegenströmung eine Strömungsgeschwindigkeit $< 0,2$ m/s hat. Bei diesen niedrigen Strömungsgeschwindigkeiten ist erfahrungsgemäß die Einhaltung konstanter Strömungsbedingungen im Strahlungsofen schwierig.

Stationäre, stabile Strömungsbedingungen kann man aber selbst bei noch kleineren Strömungsgeschwindigkeiten erreichen, wenn der Überdruck $P_2$ (gegenüber dem Atmosphärendruck) im Strahlungsofen 3 geregelt wird. Zu diesem Zweck ist der Strahlungsofen 3 etwa in seiner Mitte mit einem Drucksensor 21 ausgerüstet, der als Meßfühler Bestandteil eines Regelkreises ist. Die Regelung erfolgt in der Weise, daß der Drucksensor 21 die Abweichung $\Delta p$ von einem voreingestellten Sollwert $P_2$ feststellt und in Abhängigkeit dieser Abweichung die Drehzahl des Antriebsmotors 22 für den Abluftventilator 14 derart nachgeregelt wird, daß die Abweichung $\Delta p$ vom Sollwert minimiert wird. Mit Hilfe des Regelkreises (Sensor 21, Stellglied 22, 14) können der Druck $P_2$ im Strahlungsofen und damit auch die Strömungsbedingungen sehr genau konstant gehalten werden. Während eine direkte Messung der Strömungsgeschwindigkeit bei derart niedrigen Geschwindigkeiten praktisch nicht mehr möglich ist, erlaubt die beschriebene Druckregelung im Strahlungsofen, eine turbulenzarme Verdrängungsströmung mit einer Geschwindigkeit von nur 5 cm/s bis 10 cm/s sicher aufrechtzuerhalten.

Zur Vergleichmäßigung der Strömung am Austritt des Strahlungsofens 3 besteht die Austrittsblende 10 aus einem Laminarisierungsgewebe, das eine großflächige, druckabbauende Fläche bereitstellt. Auf diese Weise ist bereits am Ausgang des Strahlungsofens die Ausbildung der erforderlichen turbulenzarmen Gegenströmung gewährleistet. Das Laminarisierungsgewebe kann z.B. ein handelsübliches Metall-Sintergewebe sein.

In der Praxis sind normalerweise durch instationäre Betriebszustände verursachte Temperaturschwankungen im Strahlungsofen ohne besondere Vorkehrungen nicht zu vermeiden. Solche Temperaturschwankungen können zu Konvektionsströmungen im Strahlungsofen führen, die sich der verdrängungsarmen Gegenströmung überlagern und diese empfindlich stören. Der Störeinfluß ist um so größer, je niedriger die Strömungsgeschwindigkeit der Verdrängungsströmung im Strahlungsofen ist. Aus diesen Gründen ist zusätzlich eine Regelung für die Heizleistung des Strahlungsofens vorgesehen, wobei der größte Teil der Heizelemente 4 mit konstanter elektrischer Leistung betrieben wird (Grundlast) und mit dem verbleibenden Teil der Heizelemente werden die durch instationäre Betriebszustände verursachten Temperaturschwankungen weggeregelt (Regellast). Die Regellast liegt dabei im Bereich von 10% bis 20% der Gesamtlast.

Beim An- und Abfahren der Anlage und bei sonstigen instationären Betriebszuständen treten naturgemäß Störungen hinsichtlich der Strömungsbedingungen auf. Diese Störungen können minimiert werden, wenn man einmal dafür sorgt, daß die Glasbehälter 1 in dichter Packung auf dem Transportband 2 in den Strahlungsofen 3 gefahren werden und zum anderen beim Füllen und Leerfahren des Strahlungsofens die Blenden 9 und 10 am Eingang bzw. Ausgang derart nachgestellt werden, daß der freie Einströmquerschnitt auf den gleichen Wert gebracht wird wie beim Ein- und Austritt der Flaschen. Dies erleichtert das Konstanthalten des Drucks im Strahlungsofen und damit das Einhalten der turbulenzarmen Gegenströmung.

Beispiel

Standardbetriebsbedingungen für den stationären Betrieb des Strahlungsofens unter Reinraumbedingungen

$P_{LF1}$    Druck im LF-Vorhang am Einlauf I
$P_1$      Druck unterhalb der Eintrittsblende 9

$P_2$     Druck im Strahlungsofen 3
$P_3$     Druck unterhalb der Austrittsblende 10
$P_{KZ}$     Druck in der Kühlzone III oberhalb des Transportbandes 2
$P_{LF2}$     Druck im LF-Vorhang am Auslauf IV.

Sämtliche Druckangaben sind als Überdruck gegenüber dem Atmosphärendruck $P_{AT}$ zu verstehen.

| | | |
|---|---|---|
| $P_2$ | $\geq$ | 0,2 Pa |
| $P_{LF1}$ | $<$ | 2,0 Pa |
| $P_1$ | $\leq$ | $P_2$ |
| $P_3$ | $>$ | $P_2$ |
| $P_3$ | $\leq$ | $P_{KZ}$ |
| $P_{KZ}$ | $\sim$ | 4 Pa |
| $P_{LF2}$ | $\sim$ | 10 Pa |

Abmessungen des Strahlungsofens (Fig. 2 und 3)
l = ca. 3,0 m
b = ca. 1,5 m
h = ca. 0,5 m

Die Transportgeschwindigkeit des Bandes 2 betrug 16 cm/min. Durch den Wrasenabluft-Ventilator 11 wurde unter diesen Druck- und Temperaturbedingungen und bei diesen Abmessungen des Strahlungsofens ein Luftstrom von 170 m³/h abgesaugt. Im Strahlungsofen konnte damit eine Verdrängungsströmung mit einer Geschwindigkeit von ca. 7 cm/s konstant aufrechterhalten werden. Die Sterilisiertemperatur im Strahlungsofen betrug 280°C.

## Patentansprüche

1. Verfahren zur Sterilisation von temperaturbelastbaren Behältern unter Reinraumbedingungen, insbesondere Glasflaschen zur Abfüllung parenteraler Arzneimittel, bei dem die Behälter kontinuierlich über Laminar-Flow-Einheiten in einen mit Strahlungswärme arbeitenden Durchlaufsterilisator (Strahlungsofen) ein- und ausgeschleust und anschließend in der Kühlzone durch HOSCH-filtrierte Luft abgekühlt werden, wobei ein Teil der HOSCH-filtrierten Kühlluft abgezweigt und in einer turbulenzarmen Gegenströmung durch den Strahlungsofen hindurchgeleitet wird, die am Eintritt des Strahlungsofens durch einen Wrasenabluft-Ventilator abgeführt wird, dadurch gekennzeichnet, daß die Reinraumbedingungen im Strahlungsofen durch einen Überdruck $P_2$ gegenüber dem Atmosphärendruck $P_{AT}$ und eine Gegenströmung mit einer mittleren Strömungsgeschwindigkeit unterhalb 0,2 m/s aufrechterhalten werden, wobei der Überdruck $P_2$ im Strahlungsofen gemessen und mit einem Sollwert verglichen wird und der ausgangsseitige Abluftventilator für die HOSCH-filtrierte Kühlluft derart nachgeregelt wird, daß die Abweichung $\Delta p$ vom Sollwert minimiert wird, und daß der größte Teil der Strahlungsheizelemente mit konstanter elektrischer Leistung betrieben wird (Grundlast) und mit dem verbleibenden Teil der Heizelemente die durch instationäre Betriebszuzustände verursachten Temperaturschwankungen weggeregelt werden (Regellast), um im Strahlungsofen eine ungestörte, turbulenzarme Verdrängungsströmung aufrechtzuerhalten.

2. Verfahren nach Anspruch 1, dadurch gekennzeichnet, daß der Sollwert für den Überdruck $P_2$ im Strahlungsofen auf einen Wert $\geq$ 0,2 Pa eingestellt wird.

3. Verfahren nach den Ansprüchen 1 und 2, dadurch gekennzeichnet, daß die Leistung des Wrasenabluft-Ventilators am Eintritt in den Strahlungsofen so bemessen wird, daß hinsichtlich der Strömungsgeschwindigkeit im Strahlungsofen eine mittlere Reynolds-Zahl unterhalb des kritischen Wertes von 2300 eingehalten wird.

4. Verfahren nach den Ansprüchen 1 bis 3, dadurch gekennzeichnet, daß die Reinraumklasse im Strahlungsofen durch Partikelzählung mit Hilfe von rasterartig angeordneten Ansaugtulpen oberhalb der Glasbehälter laufend überwacht und dokumentiert wird.

5. Verfahren nach den Ansprüchen 1 bis 4, dadurch gekennzeichnet, daß die Sterilisationstemperatur der Glasbehälter pyrometrisch direkt gemessen wird und bei Unterschreitung der vorgegebenen Solltemperatur das Transportband so lange angehalten wird, bis die Glasbehälter auf die Sterilisationstemperatur aufgeheizt sind.

6. Verfahren nach den Ansprüchen 1 bis 5, dadurch gekennzeichnet, daß die Glasbehälter in dichter Packung auf dem Transportband durch den Strahlungsofen gefahren werden und daß beim Füllen und Leerfahren des Strahlungsofens die Blenden am Aus- bzw. Eingang derart nachgestellt werden, daß die für die stationären Reinraumbedingungen notwendigen Druck-, Strömungsgeschwindigkeits- und Temperaturwerte im Strahlungsofen weitgehend konstant bleiben.

7. Verfahren nach den Ansprüchen 1 bis 6, dadurch gekennzeichnet, daß die Temperaturverteilung über die volle Breite des Strahlungsofens durch zusätzliche, vor den Seitenwänden angeordnete Heizelemente vergleichmäßigt wird.

8. Verfahren nach den Ansprüchen 1 bis 7, dadurch gekennzeichnet, daß die Druckdifferenz zwischen Kühlzone und Strahlungsofen über ein Laminarisierungsgewebe abfällt, wodurch die in den Strahlungsofen eintretende Gegenströmung schnellstmöglich über den vollen Austrittsquerschnitt beruhigt und vergleichmäßigt wird.

## Claims

1. Method for sterilizing heat-tolerating containers under clean-room conditions, in particular glass bottles for filling with parenteral medicaments, wherein the containers are, via laminar-flow units, continuously moved into and out of a continuous sterilizer operated with radiant heat (radiant oven) and are then cooled in the cooling zone by HOSCH-filtered air, a part of the HOSCH-filtered cooling air being branched off and passed through the radiant oven in a low-turbulence counterflow which is extracted at the inlet of the radiant oven by means of a fumes/exit air fan, characterized in that the clean-room conditions are maintained in the radiant oven by a positive pressure $P_2$ as compared with atmospheric pressure $P_{AT}$ and by a counterflow of a mean flow velocity below 0.2 m/second, the positive pressure $P_2$ being measured in the radiant oven and being compared with a set value, and the exit air fan for the HOSCH-filtered cooling air on the exit side being reregulated in such a way that the deviation $\Delta p$ from the set value is minimized, and in that the major part of the radiant heater elements is operated at constant electric power (base load) and the temperature fluctuations caused by unsteady operating states are eliminated by controlling the remaining part of the heater elements (control load), in order to maintain an undisturbed, low-turbulence displacement flow in the radiant oven.

2. Method according to Claim 1, characterized in that the set value for the positive pressure $P_2$ in the radiant oven is adjusted to a value of $\geq$ 0.2 Pa.

3. Method according to Claims 1 and 2, characterized in that the power of the fumes/exit air fan at the inlet to the radiant oven is such that, with respect to the flow velocity in the radiant oven, a mean Reynolds number below the critical value of 2,300 is maintained.

4. Method according to Claims 1 to 3, characterized in that the clean-room classification in the radiant oven is continuously monitored, and recorded by means of particle counts with the aid of suction tulips in a grid-like arrangement above the glass containers.

5. Method according to Claims 1 to 4, characterized in that the sterilization temperature of the glass containers is directly measured pyrometrically and, if the temperature falls below the predetermined set value, the transport belt is stopped until the glass containers have been heated up to the sterilization temperature.

6. Method according to Claims 1 to 5, characterized in that the glass containers are moved in close packing on the transport belt through the radiant oven and, when the radiant oven is filled or emptied, the diaphragms at the inlet and outlet are readjusted in such a way that the pressure, flow velocity and temperature values required for steady clean-room conditions in the radiant oven remain largely constant.

7. Method according to Claims 1 to 6, characterized in that the temperature distribution across the full width of the radiant oven is rendered uniform by additional heater elements arranged in front of the side walls.

8. Method according to Claims 1 to 7, characterized in that the pressure difference between the cooling zone and the radiant oven decreases by a laminarization fabric, whereby the counterflow entering the radiant oven is rendered quiet and uniform as quickly as possible across the full exit cross-section.

## Revendications

1. Procédé de stérilisation de récipients pouvant résister à la température, notamment de flacons en verre destinés à être remplis de médicaments à absorbtion par voie parentérale, dans des conditions de salle blanche, selon lequel, par l'intermédiaire d'unités à écoulement laminaire, on introduit les récipients d'une manière continue, par un sas, dans un stérilisateur à traversée continue, fonctionnant par chaleur radiante (four radiant) et on les en fait sortir, par un sas, puis on les refroidit, dans la zone de refroidissement, au moyen d'air filtré par un filtre à rendement élevé pour matières en suspension dans l'air, ou filtre REMSA, une partie de l'air de refroidissement, filtré sur filtre REMSA, étant déviée et dirigée dans le four radiant suivant un contre-courant peu turbulent, cette partie de l'air de refroidissement étant évacuée à l'entrée du four radiant par un ventilateur de buées, caractérisé en ce que les conditions de salle blanche sont maintenues dans le four radiant au moyen d'une surpression ($P_2$), par rapport à la pression atmosphérique ($P_{AT}$), et d'un contre-courant qui a une vitesse moyenne d'écoulement inférieure à 0,2 m/s, cette surpression ($P_2$) régnant dans le four radiant étant mesurée et comparée à une valeur de consigne et le ventilateur d'extraction disposé du côté de la sortie pour l'air de refroidissement filtré sur filtre REMSA faisant alors l'objet d'une régulation telle que l'écart $\Delta p$ par rapport à la valeur de consigne soit rendu minimal, et en ce qu'on fait fonctionner à puissance électrique constante (charge de base) la plus grande partie des éléments de chauffage par rayonnement et, au moyen des éléments de chauffage restants, on réalise une régulation éliminant les fluctuations de température dues à des conditions non-stationnaires de fonctionnement (charge de régulation), afin de maintenir dans le four radiant un écoulement en reflux, peu turbulent, qui n'est pas perturbé.

2. Procédé suivant la revendication 1, caractérisé en ce qu'on règle à une valeur $\geq$ 0,2 Pa la valeur de consigne prévue pour la surpression ($P_2$) régnant dans le four radiant.

3. Procédé suivant l'une des revendications 1 et 2, caractérisé en ce qu'on mesure la puissance du ventilateur de buées, situé à l'entrée dans le four radiant, de telle façon qu'en ce qui concerne la vitesse d'écoulement dans le four radiant, qui offre une surface libre de 0,7 $m^2$ environ, on maintient un nombre de Reynolds moyen, inférieur à la valeur critique de 2300.

4. Procédé suivant l'une des revendications 1 à 3, caractérisé en ce qu'on surveille et analyse d'une manière continue la classe de salle blanche, qui se présente dans le four radiant, en comptant les particules au moyen de tulipes d'aspiration disposées en forme de réseau au-dessus des récipients en verre et pouvant être branchées de manière successive sur un compteur de particules.

5. Procédé suivant l'une des revendications 1 à 4, caractérisé en ce qu'on mesure directement, par voie pyrométrique, la température de stérilisation des récipients en verre et, en cas de dépassement vers le bas de la température de consigne fixée à l'avance, on maintient la bande de transport à l'arrêt jusqu'à ce que les récipients en verre soient portés à la température de stérilisation.

6. Procédé suivant l'une des revendications 1 à 5, caractérisé en ce qu'on fait passer les récipients en verre dans le four radiant d'une manière serrée les uns par rapport aux autres sur la bande de transport et en ce que, lorsqu'on remplit le four radiant et lorsqu'on le vide, on règle alors les obturateurs prévus à la sortie et à l'entrée d'une façon telle que les valeurs de pression, de vitesse d'écoulement et de température, se présentant dans le four radiant, qui sont nécessaires pour les conditions stationnaires de salle blanche restent extrêmement constantes.

7. Procédé suivant l'une des revendications 1 à 6, caractérisé en ce qu'on uniformise la répartition de température sur toute la largeur du four radiant au moyen d'éléments supplémentaires de chauffage disposés devant les parois latérales.

8. Procédé suivant l'une des revendications 1 à 7, caractérisé en ce que la différence de pression entre la zone de refroidissement et le four radiant est réduite par l'intermédiaire d'une toile rendant l'écoulement laminaire, de sorte que le contre-courant se produisant dans le four radiant se stabilise et s'uniformise de la manière la plus rapide possible sur toute la section transversale de la sortie.

FIG. 1

FIG. 2

FIG. 3